(19) Europäisches Patentamt
European Patent Office
Office européen des brevets

(11) Publication number: **0 281 326 A2**

(12) # EUROPEAN PATENT APPLICATION

(21) Application number: 88301647.9

(22) Date of filing: 25.02.88

(51) Int. Cl.4: C07C 69/07 , C07C 67/08 , C07C 67/293 , C07C 67/11 , C07C 13/43 , C07C 13/42 , //C07C33/12

(30) Priority: 05.03.87 GB 8705118
26.03.87 GB 8707302
26.03.87 GB 8707310

(43) Date of publication of application: 07.09.88 Bulletin 88/36

(84) Designated Contracting States: BE DE FR GB IT NL

(71) Applicant: The British Petroleum Company p.l.c.
Britannic House Moor Lane
London EC2Y 9BU(GB)

(72) Inventor: Butters, Michael The British Petroleum Com. p.l.c.
Chertsey Road
Sunbury-on-Thames Middlesex TW16 7LN(GB)

(74) Representative: Scott, Susan Margaret et al
BP INTERNATIONAL LIMITED Patents & Agreements Division Chertsey Road
Sunbury-on-Thames Middlesex TW16 7LN(GB)

(54) **Norbornadiene derivatives.**

(57) Novel compounds of the general formula:-

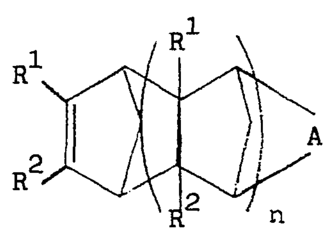

(I)

in which each of $R^1$ and $R^2$ independently represents a hydrogen atom or an alkyl, cycloalkyl or aryl group; n is 0 or an integer from 1 to 4; and A is an optionally substituted propenyl formate group of the general formula:

$R^3 - \underset{|}{C} = \underset{|}{C} - CR^4R^5 - O.CHO$ (II) or $CR^4R^5 = \underset{|}{C} - \underset{|}{C}R^3 - O.CHO$ (III)

in which $R^3$ is a hydrogen atom or an alkyl, cycloalkyl or aryl group; and each of $R^4$ and $R^5$ independently

EP 0 281 326 A2

represents a hydrogen atom or an alkyl, cycloalkyl or aryl group, or $R^4$ and $R^5$ together represent an alkylene chain; are useful chemical intermediates. They may be converted by an internal hydrogenolysis reaction into known norbornene derivatives.

## NORBORNADIENE DERIVATIVES

The present invention relates to novel norbornadiene derivatives, to a method of preparing them, and their use.

Norbornene derivatives are versatile chemical intermediates, and have a variety of uses. For example, methylene norbornene is a useful chemical intermediate and has been used as a cross-linking agent in rubbers especially in ethylene-propylene-diene (the so-called "EPD") rubbers. These monomers have hitherto been produced either by the Diels Alder addition of propadiene to cyclopentadiene or by the addition of methyl acetylene to cyclopentadiene followed by isomerisation to produce methylene norbornene. Another method used to produce these monomers includes a two stage process in which allyl alcohol ester or halide is added to cyclopentadiene followed by elimination of acid or dehydrohalogenation respectively to form methylene norbornene. Further methods used hitherto include a two-stage process in which vinyl acetate is added to cyclopentadiene followed by hydrolysis of the resultant adduct to form an alcohol which is then oxidised by chromium trioxide to a ketone and then reacted with a phosphomethylene derivative so as to form a -C=C-bond and hence methylene norbornene.

In all the above cases, the principal disadvantage is one of low yield of the product combined with relatively high reaction temperatures, e.g. above 160°C during the final step. In addition, the high reaction temperatures may result in undesirable decomposition isomerisation or polymerisation of the desired reaction product and may aggravate any corrosive effects of the reactant or product halides or metal hydroxides.

It is an object of the present invention to mitigate these problems by starting from novel hydrocarbyl formate esters.

Accordingly the present invention provides a compound of the formula:-

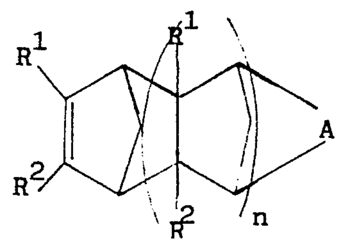

(I)

in which each of $R^1$ and $R^2$ independently represents a hydrogen atom or an alkyl, cycloalkyl or aryl group; n is 0 or an integer from 1 to 4; and A is an optionally substituted propenyl formate group of the general formula:

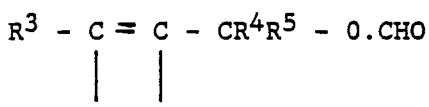

(II)

or

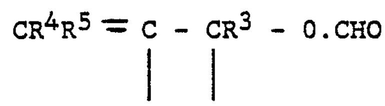

(III)

in which $R^3$ is a hydrogen atom or an alkyl, cycloalkyl or aryl group; and each of $R^4$ and $R^5$ independently represents a hydrogen atom or an alkyl, cycloalkyl or aryl group, or $R^4$ and $R^5$ together represent an alkylene chain.

Except here otherwise stated, throughout this specification and claims, any alkyl group preferably has up to 6, especially up to 4, carbon atoms; a cycloalkyl group preferably has 5 or 6 carbon atoms; an aryl group is preferably a phenyl group; and an alkylene chain is preferably a tetra-or penta-methylene chain.

Preferably each of $R^1$, $R^2$, $R^3$, $R^4$ and $R^5$ independently represents a methyl group or a hydrogen atom. Most preferably, each of $R^1$, $R^2$ and $R^3$ represents a hydrogen atom, and each of $R^4$ and $R^5$ independently represents a hydrogen atom or a methyl group. Preferably n is 0 or an integer from 1 to 3, most preferably 0.

It will be appreciated that compounds of the general formula I in which A is group II or group III, are structural isomers of each other. The process according to the invention usually produces a mixture of the

two isomers. The relative proportions of the two isomers in the reaction mixture can of course be varied, if desired, by varying the reaction conditions.

The invention also provides a process for the preparation of a compound of the general formula I, which comprises either:

(i) esterifying an alcohol of the general formula:

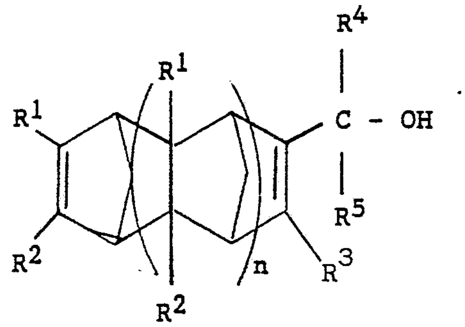

(IV)

using any suitable formylating agent;

or

(ii) reacting a compound of the general formula:

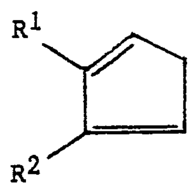

(V)

with a compound of the general formula:

$R^3$ -C ≡ C - $CR^4R^5$ -0.CHO (VI)

or

(iii) reacting a compound of the general formula:

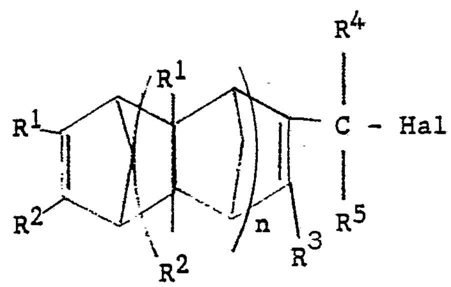

(VII)

in which Hal is a halogen atom, with an alkali metal formate, preferably sodium formate, under phase transfer conditions.

Any suitable formylating agent may be used for reaction (i). Typical reagents include formic acid; a mixed anhydride of formic acid and an alkanoic acid, for example acetic acid; and esters of formic acid, for example, methyl or butyl formate.

When formic acid is used, this is optionally mixed with acetic anhydride, and a catalyst may be present. A pyridine catalyst leads predominantly to a compound of formula I in which A represents group II, whereas the use of a phosphoric acid catalyst increases the proportion of the compound of formula I in which A represents group III. The molar ratio of acetic anhydride to formic acid is suitably from 5:1 to 0.2:1, preferably from 2:1 to 0.5:1. A reaction temperature of from 0 to 100°C, preferably from 0 to 50°C if using a pyridine catalyst, is preferred, and atmospheric pressure is convenient.

In reaction (i) it should be noted that the nature of the substituents $R^4$ and $R^5$ in formula (IV) and the esterification conditions chosen may be used to control the type of product produced. For instance, if in formula (IV)

(a) each of $R_4$ and $R_5$ is H then by using relatively mild esterification conditions e.g. a formic-acetic anhydride mixture at 20°C, the product is predominantly a formate ester of formula (I) in which A is group (II);

(b) either $R^4$ or $R^5$ is a $CH_3$-group then using the same esterification conditions as in (a) above, the product is a mixture of compounds of the formula (I) in which A is group (II) and (III);

(c) both $R^4$ and $R^5$ are $CH_3$-groups then under the same esterification conditions as in (a) above the resultant product is predominantly the compound of formula I in which A is group III.

In all cases, using relatively stronger esterification conditions than those in (a) above results in a product containing increasing amounts of compounds of the formula I in which A is group III.

Thus, if a product containing relatively pure compound of formula I in which A is group II is desired, it is preferable to use mild esterification conditions.

The compound of general formula (IV) may be prepared by reacting a compound of the general fromula (V) with a compound of the general formula:

$R^3\text{-}C \equiv C\text{-}C\,R^4R^5OH$ (VIII)

This reaction may be carried out at elevated temperature and pressure by the method described in Tetrahedron, 41, 749, (1985). The molar ratio of compound V to compound VIII can be varied; by using an appropriate ratio, compounds of formula IV with varying values of n can be prepared.

The reaction of the compounds V and VI may be carried out under the same conditions as those described above for the reaction of the compounds V and VIII.

Compounds of the general formula VI may be prepared by known methods, for example by formulation of the corresponding alcohol VIII using formic acid, a mixed anhydride of formic acid and an alkanoic acid, or an alkyl formate.

The compound of the general formula VII may be prepared under known conditions by reaction of a compound of formula (V) with a compound of the general formula:

$R^3\text{-}C \equiv C\text{-}CR^4C^5\text{-Hal.}$ (IX)

Various compounds of the general formulae I and IV have been characterised by the following physical characteristics:-

Methylene Norbornene formate

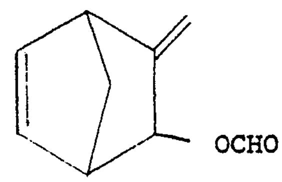

| Elemental | $^{13}$Cnmr Delta Values [$CDCl_3$ solvent] | $^{1}$Hnmr Delta Values | Mass Spectrum mass/charge | Boiling Point |
|---|---|---|---|---|
| C 72.3% | 161 | 8.1 | 39 (61%) | ca 60°C/666 Pa |
| H 6.8% | 150 | 6.25 (mult) | 51 (31%) | |
| O 21.9% | 140 | 6.05 (mult) | 66 (100%) | |
| | 134 | 5.1 | 77 (70%) | |
| | 108 | 5.05 | 78 (80%) | |
| | 75 | 5.0 | 79 (59%) | |
| | 49 | 3.15 | 91 (70%) | |
| | 48 | 2.9 | 104 (90%) | |
| | 47 | 1.8 | 122 (30%) | |
| | | | 150 (14%) | |

2-(1'-hydroxyethyl) Norbornadiene

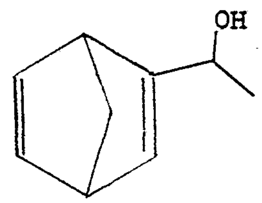

| $^{1}$Hnmr Delta values | $^{13}$Cnmr Delta values | Mass Spectrum mass/charge |
|---|---|---|
| 6.4 | 161 (doublet) | 39 (34%) |
| 5.9 | 142.5 (quartet) | 43 (46%) |
| 4.0 | 134 (doublet) | 45 (20%) |
| 3.25 | 73 (doublet) | 65 (31%) |
| 3.1 | 66 (doublet) | 66 (100%) |
| 3.05 | 50 (quartet) | 67 (22%) |
| 1.6 | 20.5 | 77 (31%) |
| 0.8 | | 79 (25%) |
| | | 91 (54%) |
| | | 93 (40%) |
| | | 136 (19%) |

Norbornadiene-1',1'-dimethylcarbinol

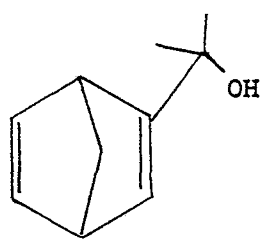

| $^{1}$Hnmr | $^{13}$Cnmr | Mass Spectrum |
|---|---|---|
| Delta values | | mass/charge |
| 6.8 (doublet) | 166 | 43 (27%) |
| 6.35 | 145 | 59 (13%) |
| 4.9 | 134 | 66 (100%) |
| 3.6 | 74 | 79 (14%) |
| 2.0 | 72 | 91 (34%) |
| 1.35 | 50 (mult) | 117 (34%) |
| | 28 | 132 (22%) |
| | | 150 (24%) |

Ethylidene Norbornene formate (cis + trans mixture)

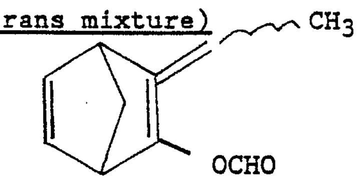

| $^{13}$Cnmr | $^{1}$Hnmr | Mass Spectrum |
|---|---|---|
| Delta values | | mass/charge |
| 161 | 8.1 | 66 (100%) |
| 140.5 | 6.3 | 77 (31%) |
| 139.5 | 6.1 | 79 (41%) |
| 133.5 | 5.5 | 91 (66%) |
| 118 | 5.15 | 107 (30%) |
| 75 | 3.45 | 117 (56%) |
| 47 | 2.9 | 118 (44%) |
| 46 | 1.7 | 136 (39%) |
| 43 | 1.3 | 164 (22%) |
| 14 | | |

Iso-propylidene norbornene formate

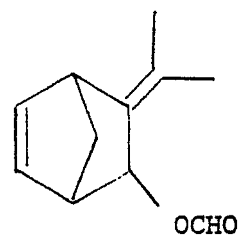

| $^1$Hnmr | $^{13}$Cnmr | Mass Spectrum |
|---|---|---|
| Delta values | | mass/charge |
| 8.15 | 160 | 66 (100%) |
| 6.4 | 140 | 79 (31%) |
| 6.1 | 133 | 91 (58%) |
| 5.3 | 126 | 117 (68%) |
| 3.5 | 75 | 122 (33%) |
| 3.0 | 48 | 132 (49%) |
| 2.1 | 47 | 149 (29%) |
| 1.9 | 44 | 178 (38%) |
| 1.7 (2 peaks) | 21 | |

Norbornadiene Methyl Formate

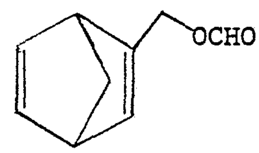

| Elemental | $^{13}$Cnmr | $^1$Hnmr | Mass Spectrum | Boiling Point |
|---|---|---|---|---|
| | Delta Values [$CDCl_3$ solvent] | | mass/charge | |
| C72.3% | 162 | 8.1 | 39 (61%) | 60°C/666 Pa |
| H 6.8% | 152 | 6.8 | 51 (31%) | |
| O 21.9% | 144 | 6.75 | 66 (100%) | |
| | 143 | 4.8 | 77 (70%) | |
| | 141 | 3.55 | 78 (80%) | |
| | 74 | 2.0 (mult) | 79 (59%) | |
| | 62 | | 91 (70%) | |
| | 52 | | 104 (90%) | |
| | 50 | | 122 (30%) | |
| | | | 150 (14%) | |

| $^1$Hnmr | $^{13}$Cnmr | Mass Spectrum |
|---|---|---|
| Delta values | | mass/charge |
| 6.64 | 161 (doublet) | 39 (34%) |
| 6.14 | 142.5 (quartet) | 43 (46%) |
| 4.24 | 134 (doublet) | 45 (20%) |
| 3.49 | 73 (doublet) | 65 (31%) |
| 3.34 | 66 (doublet) | 66 (100%) |
| 3.29 | 50 (quartet) | 67 (22%) |
| 1.84 | 20.5 | 77 (31%) |
| 1.04 | | 79 (25%) |
| | | 91 (54%) |
| | | 93 (40%) |
| | | 136 (19%) |

2-(Ethyl-1'-formate) norbornadiene

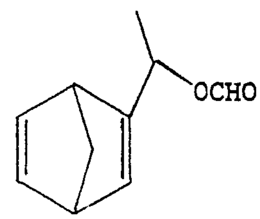

| $^1$Hnmr | $^{13}$Cnmr | Mass Spectrum |
|---|---|---|
| Delta values | | mass/charge |
| 8.1 | 160 | 66 (100%) |
| 6.8 (2 peaks) | 156 (2 peaks) | 77 (31%) |
| 6.5 | 142-143 (2 peaks) | 79 (41%) |
| 5.7 | 137.5 (2 peaks) | 91 (66%) |
| 3.5 (2 peaks) | 73 (2 peaks) | 107 (30%) |
| 1.3 | 68.5 (2 peaks) | 117 (56%) |
| | 50 (2 peaks) | 118 (44%) |
| | 18 (2 peaks) | 186 (39%) |
| | | 164 (22%) |

The invention further provides a process for the preparation of a compound of the general formula X and/or XI

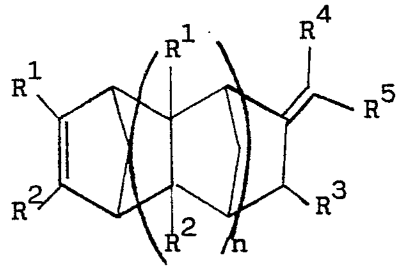

(X)

9

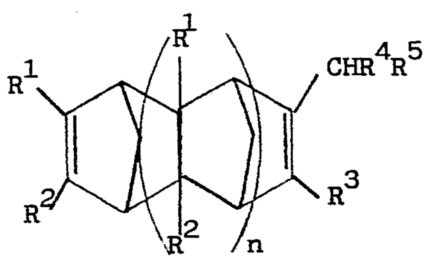

(XI)

which comprises heating a compound of the general formula I in the presence of a hydrogenolysis catalyst. The catalyst may be homogenous or heterogenous, and preferably comprises a complex catalyst of a Group VIB or Group VIII metal, for example palladium, platinum, ruthenium, rhodium, iridium, cobalt, molybdenum and tungsten. The use of palladium is preferred.

The catalyst may comprise a metal salt, for example a carboxylate or a halide, such as palladium acetate or rhodium chloride, together with a promoter, for example an alkyl or aryl phosphine or diphosphine, preferably triphenylphosphine, $PPh_3$. Alternatively, performed catalysts may be used. Such catalyst include the following: $RcCl_2(PPh_3)_3$; $PtHNO_3(PPh_3)_2$; $Cl_2Ni(PPh_3)_2$; $RhCl_2(CO)_2$; $RhCl(PPh_3)_3$; Ir-(cycloocta-1,5-diene)$(PCH_3Ph_2)_2.PF_6$; $Co_2(CO)_8$; $RhH(CO)(PPh_3)_3$. Such catalysts may also be used in their hydride form, which may be generated in situ.

Suitable heterogenous catalysts may be prepared by supporting suitable metals or metal compounds on a solid support, for example silica, alumina, a zeolite, or a polymer such as polystyrene.

The reaction temperature is preferably in the range of from 50 to 150°C.

It is preferable to carry out the hydrogenolysis in a solvent, preferably a polar solvent such as for example N-methyl pyrrolidone, N,N-di methyl acetamide or the monomethyl ether of triethylene glycol. These solvents can give a high proportion of the preferred compound. However, the solvent constraints are not decisive because compound XI is readily transformed into compound X, which is usually the preferred product of the reaction, by conventional acidic or basic isomerisation catalysts, e.g. the H form of zeolites notably H form of zeolite Y.

Accordingly a process for the preparation of a compound of the general formula X may if desired comprise the additional step of admixing a resultant reaction mixture containing compounds X and XI with a catalyst capable of isomerising a compound XI to give a compound X.

The hydrogenolysis reaction can be carried out batchwise or continuously. In the continuous process in particular, it is possible to steadily introduce into a reactor under vacuum containing the catalyst a solution of diene or hydrocarbylidene formate at one end and to continuously withdraw the product hydrocarbon and the eliminated carbon dioxide from the other end of the same reactor. The difference in the boiling points between the reactant diene formate and the product facilitates such a continuous reaction.

The present invention provides a cheap and convenient route to a range of compounds which are rather difficult to synthesis by other means. Thus, for example methylene norbornene may be prepared in good yield by using cyclopentadiene and propargyl alcohol as starting materials to prepare the compounds of formula I in which n is 0 and each of $R^1$, $R^2$, $R^3$, $R^4$ and $R^5$ is hydrogen, in two steps; internal hydrogenolysis to produce the corresponding compound of formula X possibly in admixture with the compound of formula XI; and if desired, a further isomerisation step to convert compound XI into compound X, methylene norbornene.

The present invention is further illustrated with reference to the following Examples.

## Example 1

Propargyl alcohol 112g (2 moles) was mixed with cyclopentadiene 66g (1 mole) and 1.1g (0.01 moles) of hydroquinone (inhibitor). The mixture was then heated in an autoclave to 175°C for 14 hours. The product mixture was then distilled and the first fraction contained propargyl alcohol 56g (1 mole) which could be re-used in a further reaction. A further fraction (boiling point ca 65°C/133 Pa) was collected and shown to be norbornadiene methyl alcohol (ca 60% conversion based on cyclopentadiene).

Example 2 - Esterification of Norbornadiene Methyl Alcohol

(a) Norbornadiene methyl alcohol 122g (1 mole) and phosphoric acid (1%) were mixed with formic acid 55.2g (1.2 moles) at 20°C and the mixture stirred for 25 hours. The excess acid was then neutralized by addition of sodium carbonate saturated solution. The organic layer was separated and then the diene formate product purified by distillation (boiling point ca. 60°C/666 Pa) and shown to consist of norbornadiene methyl formate and methylene norbornene formate mixture.

(b) Norbornadiene methyl alcohol 122g (1 mole) and pyridine 3.95g (0.05 mole) were mixed with a formic-acetic mixture (FAM) (FAM:87.4g (1.9 mole) of formic acid was mixed with 122.4g (1.2 moles) of acetic anhydride and warmed at 50°C for 1 hour before being cooled to 20°C) at 20°C and the mixture stirred for 5 hours. The excess acid was then neutralized by addition of sodium carbonate saturated solution. The organic layer was separated and then the diene formate product purified by distillation (boiling point ca. 60°C/666 Pa) and shown to consist of norbornadiene methyl formate ca. 100% conversion).

(c) Norbornadiene methanol 122g (1 mole), methyl formate 6kg (100 mole) dibutyl tin oxide (0.05 mole), and methylene blue (0.01 mole) were mixed in a glass flask and heated under reflux. The methanol produced was co-distilled with methyl formate and allowed to run through a column of zeolite 4A pellets, the methyl formate being returned to the reaction mixture.

After 6 hours, the methyl formate was removed at 34°C under atmospheric pressure, and the product was purified by distillation. Norbornadiene methyl formate was obtained in 80% yield.

Example 3 - Hydrogenolysis of Norbornadiene Methyl Formate Methylene Norbornene formate

A catalyst system composed of 11.22g (0.05 moles) of palladium acetate and 104.79g (0.4 moles) of triphenyl phosphine in dimethylacetamide solvent (120 mls) was warmed to 80°C under reduced pressure (26.7 kPa). A total charge of 150g (1 mole) norbornadiene methyl formate/methylene norbornene formate was slowly added (dropwise) into the top of the catalyst/solvent reactor, and after 5 minutes induction period, a steady stream of carbon dioxide, methyl norbornadiene and methylene norbornene began to distil out of the catalyst pot down a condensor into a collector. At the end of the reaction sequence, the collector contained a near quantitative conversion of the diene formate into a 2:1 mixture of methylene norbornene and methyl norbornadiene.

Example 4 - Isomerisation of a Mixture of Methylene Norbornene and Methylnorbornadiene, as produced by Hydrogenolysis of Norbornadiene Methyl Formate/Methylene norbornene formate into Methylene Norbornene.

A product mixture of methyl-norbornadiene and methylenenorbornene removed from a continuous reactor system by (80°C/reduced pressure) was mixed with 5% (by weight) of $H^+$ zeolite Y (precalcined at 550°C/15 hours) and the mixture heated to 80°C for 1½ hours. After this period, the mixture had virtually completely isomerized to methylene norbornene (ca. 100%). A similar result was achieved by performing the isomerisation by passing the mixture of alkenes through a heated-packed column of $H^+$ zeolite Y.

Example 5

2-Methyl-3-butyn-2-ol 168g (2 moles) was mixed with cyclopentadiene 66g (1 mole) and 1.1g (0.01 moles) of hydroquinone (inhibitor). The mixture was then heated in an autoclave at 175°C for 14 hours. The product mixture was then distilled, the first fraction contained 2-methyl-3-butyn-2-ol and a further fraction gave a mixture of dicyclopentadiene and norbornadiene-1′,1′-diemthyl carbinol which was separated by a further fractional distillation.

Example 6 - Esterification

Norbornadiene-1',1'-dimethyl carbinol 150g (1 mole) and pyridine 3.95g (0.05 moles) were mixed with formic-acetic mixture (FAM:87.4g (1.9 moles) of formic acid was mixed with 122.4g (1.2 moles) of acetic anhydride and warmed to 50°C, then cooled back to 20°C) at 20°C and the mixture was allowed to react for 12 hours. Excess acid was neutralised with sodium carbonate and the formate product purified by distillation (Bpt ca 66°C/86.7 Pa) and shown by $^{13}C$/Hnmr and mass spectroscopy to consist of a mixture of norbornadiene-1',1'-dimethyl carbinyl formate and iso-propylidene norbornene formate (major isomer).

Example 7 - Hydrogenolysis of Norbornadiene-1',1'-dimethyl Carbinol Formate Product

A catalyst system as described in Example 3 was used to convert 178g (1 mole) of norbornadiene-1',1'-dimethyl carbinol formate product mixture into isopropylidene norbornene and 2-isopropyl norbornadiene. This mixture was isomerised into isopropylidene norbornene using the procedure given in Example 4.

Example 8

D,L-3-butyn-2-ol 140g (2 moles) was mixed with cyclopentadiene 66g (1 mole) and 1.1g (0.01 moles) of hydroquinone (inhibitor). The mixture was then heated in an autoclave to 175°C for 14 hours. The product mixture was distilled, and the first fraction contained 3-butyn-2-ol 70g (1 mole) which could be re-used in a further reaction. A second fraction contained 2-(1'-hydroxyethyl)norbornadiene (boiling point ca 94°C/2.67 MPa) in about 55% yield.

Example 9

2-(1'-hydroxyethyl)norbornadiene 136g (1 mole) was mixed with FAM (as described for norbornadiene-1',1'dimethyl carbinol in Example 6 and mixture stirred for 5 hours. The excess acid was neutralized by addition of sodium carbonate saturated solution. The organic layer was separated and then the formate product purified by distillation (boiling point ca 60°C/66.7 Pa) and shown to consist of 2-(ethyl-1'-formate) norbornadiene and ethylidene norbornene formate.

Example 10

A catalyst system as described in Example 3 was used to convert 164g (1 mole) of 2-(ethyl-1'-formate) norbornadiene/ethylidene norbornene formate into a mixture (3:1) of 2-ethyl norbornadiene and ethylidene norbornene (mixture of cis and trans isomers). This diene product mixture was converted to ca 100% ethylidene norbornene using the procedure as described in Example 4 (for methylene norbornadiene).

**Claims**

1. A compound of the formula:-

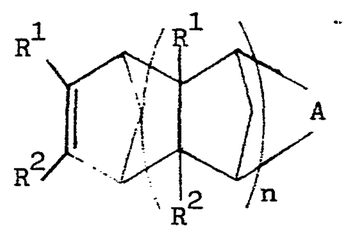

(I)

in which each of $R^1$ and $R^2$ independently represents a hydrogen atom or an alkyl, cycloalkyl or aryl group; n is 0 or an integer from 1 to 4; and A is an optionally substituted propenyl formate group of the general formula:

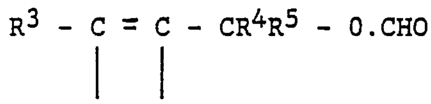 or 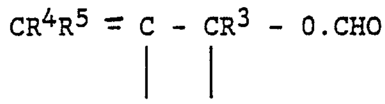

(II) (III)

in which $R^3$ is a hydrogen atom or an alkyl, cycloalkyl or aryl group; each of $R^4$ and $R^5$ independently represents a hydrogen atom or an alkyl, cycloalkyl or aryl group, or $R^4$ and $R^5$ together represents an alkylene chain.

2. A compound as claimed in claim 1, in which each of $R^1$, $R^2$, $R^3$, $R^4$ and $R^5$ independently represents a methyl group or a hydrogen atom.

3. A compound as claimed in claim 2, in which each of $R^1$, $R^2$ and $R^3$ represents a hydrogen atom, and each of $R^4$ and $R^5$ independently represents a hydrogen atom or a methyl group.

4. A compound as claimed in any one of claims 1 to 3, in which n is 0.

5. A process for the preparation of a compound as claimed in claim 1, which comprises either:

(i) esterifying an alcohol of the general formula:

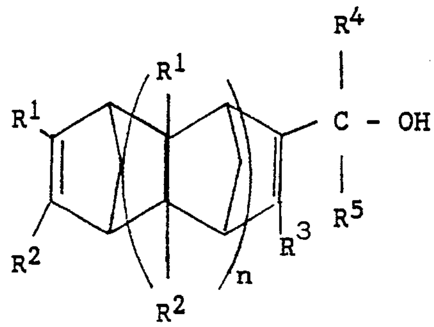

(IV)

using an suitable formylating agent;

or

(ii) reacting a compound of the general formula:

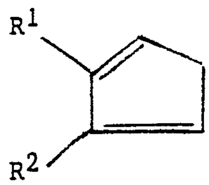

(V)

with a compound of the general formula:

$R^3$ -C ≡ C - $CR^4R^5$ -0.CHO (VI)

or

(iii) reacting a compound of the general formula:

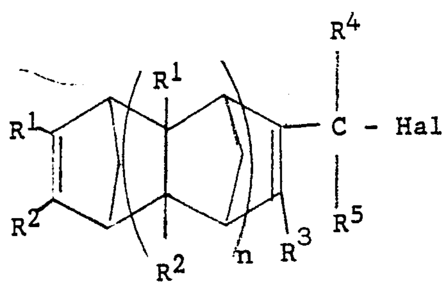

(VII)

in which Hal is a halogen atom, with an alkali metal formate under phase transfer conditions.

6. A process for the preparation of a compound of the general formula X and/or XI

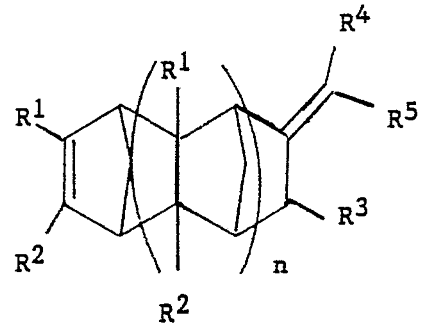

(X)

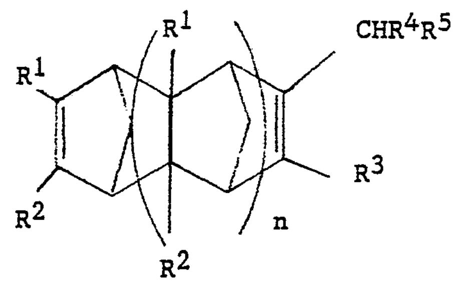

(XI)

which comprises heating a compound of the general formula I in the presence of a hydrogenolysis catalyst.

7. A process as claimed in claim 6, which comprises the additional step of admixing a resultant reaction mixture contacting compounds X and XI with a catalyst capable of isomerising a compound XI to give a compound X.